(19) Europäisches Patentamt
European Patent Office
Office européen des brevets

(11) Veröffentlichungsnummer: **0 054 676**
**B1**

(12) # EUROPÄISCHE PATENTSCHRIFT

(45) Veröffentlichungstag der Patentschrift: **02.11.83**

(51) Int. Cl.³: **C 12 Q 1/66**, G 01 N 33/50, C 12 Q 1/48

(21) Anmeldenummer: **81108755.0**

(22) Anmeldetag: **22.10.81**

(54) **Verfahren zur Bestimmung von HL-Antigenen.**

(30) Priorität: **18.12.80 DE 3047860**

(43) Veröffentlichungstag der Anmeldung: **30.06.82 Patentblatt 82/26**

(45) Bekanntmachung des Hinweises auf die Patenterteilung: **02.11.83 Patentblatt 83/44**

(84) Benannte Vertragsstaaten: **AT BE CH DE FR GB IT LI LU NL SE**

(56) Entgegenhaltungen:
**DE - A - 2 823 816**
**GB - A - 1 330 594**

**CHEMICAL ABSTRACTS, Band 92, Nr. 21, 26. Mai 1980, Seite 294, Nr. 177041q Columbus, Ohio, U.S.A. B. DESCAMPS: "*Determination of intracellular adenosine triphosphate for detecting anti-HLA antibody-mediated cytolysis. Introduction to a new method for HLA typing*"**
**CHEMICAL ABSTRACTS, Band 93, Nr. 7, 18. August 1980, Seite 440, Nr. 65272e Columbus, Ohio, U.S.A. F.J. FEHRENBACH et al.: "*Measurement of bacterial cytolysins with a highly sensitive kinetic method*"**
**NATURE, Band 204, 5. Dezember 1964, Seiten 998-1000 PAUL I. TERASAKI et al.: "Microdroplet Assay of Human Serum Cytotoxins"**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Scherer, Reiner, Dr., Connollystrasse 22/III, D-8000 München 40 (DE)**
Erfinder: **Wulff, Karl, Dr., Pütrichstrasse 18, D-8120 Weilheim (DE)**
Erfinder: **Treffert, Christine, Staudenmoosstrasse 5, D-8132 Tutzing-Unterzeismering (DE)**

(74) Vertreter: **Weickmann, Heinrich, Dipl.-Ing. et al, Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22, D-8000 München 86 (DE)**

**EP 0 054 676 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Bestimmung von HL-Antigenen

Die Erfindung betrifft ein Verfahren zur Bestimmung von HL-Antigenen auf Lymphozyten.

Humanleukozyten-Antigene (HL-Antigene) sind Antigene des Histokompatibilitätssystems. Sie sind verantwortlich für die Erkennung fremden Gewebes durch den Körper und haben daher eine grosse Bedeutung für Organtransplantationen und Bluttransfusionen, bei denen Leukozyten mit übertragen werden. HL-Antigene sind Glykoproteine auf der Zelloberfläche von kernhaltigen Zellen und Thrombozyten. Sie lassen sich am einfachsten auf Lymphozyten nachweisen. Neben ihrer Bedeutung als Unverträglichkeitsfaktor bei Transplantationen, nehmen sie auch Einfluss auf den Ablauf lebensbestimmender biologischer Prozesse, wie die immunologische Erkennung und Immunantwort, sowie Empfänglichkeit für Erkrankungen.

Man unterscheidet nach ihren Genarten drei Gurppen von HL-Antigenen.

1) Die serologisch definierten Antigene (HLA-A, HLA-B, HLA-C);

2) die lymphozytär definierten Antigene (HLA-D) und

3) die B-zelldefinierten Antigene (HLA-DR).

Die erste und dritte Antigengruppe kann serologisch mit Hilfe spezifischer Antiseren nachgewiesen werden. Die HLA-D-Antigene sind nicht serologisch bestimmbar, jedoch sind sie so stark mit den serologisch definierten HLA-DR-Antigenen assoziiert, dass eine Identität nicht ausgeschlossen wird. Zur Zeit sind rund 80 verschiedene HLA-Merkmale serologisch charakterisiert.

Die serologische Bestimmung von HL-Antigenen auf Lymphozyten hat eine grosse Bedeutung in der Medizin vor allem auf folgenden Gebieten erlangt:

1) Transplantation

Es wurde gefunden, dass die Überlebenszeit eines Transplantats von einem mit dem Empfänger verwandten Spender grösser ist, wenn Spender und Empfänger identische HL-Antigene besitzen, d.h. die Gewebeverträglichkeit hängt von der Ähnlichkeit des HLA-Systems von Spender und Empfänger ab. Für Organtransplantationen und auch für die Transfusion von Leukozyten, Thrombozyten und anderen Blutbestandteilen ist daher eine genaue Kenntnis der HL-Antigene von Spender und Empfänger erforderlich.

2) Diagnose genetisch determinierter Krankheiten

Bestimmte HL-Antigenmuster sind mit einer Reihe verschiedenartiger Erkrankungen assoziiert, z.B. mit immunpathologischen Erkrankungen, primär-chronischer Polyarthritis, Coeliakie, Myasthenia gravis, Reitersyndrom, chronischer Hepatitis und möglicherweise auch Leukämie. Durch Bestimmung der HL-Antigene ist die Möglichkeit für eine Frühdiagnose gegeben, so dass eine gezielte Therapie bereits vor Erscheinen der Symptome angewandt werden kann.

3) Vaterschaftsgutachten

Aufgrund der Vielzahl der Merkmale und des dominanten Erbgangs ist das HLA-System sehr gut für die Paternitätsserologie geeignet. Durch die Berücksichtigung von 21 HLA-Merkmalen konnte die Ausschlusswahrscheinlichkeit für Nichtväter bei Vaterschaftsgutachten auf 98,5% erhöht werden.

Es ist bekannt, HL-Antigene auf Lymphozyten mit Hilfe des Zytotoxizitätstests nach P.I. Terasaki und J.D. McClelland [Nature 204, 998-1000 (1964)] und S. Kissmeyer-Nielsen und K.E. Kjerbye (Histocompatibility Test. 1967, 381-383, E.S. Curtoni, P.L. Mattiuz, R.M. Posi, Ed. Munks Gaard, Kopenhagen 1967) zu bestimmen. Als Testzellen werden Lymphozyten verwendet, die durch Ficoll-Isopaque-Gradientenzentrifugation aus venösem Blut gewonnen werden. Die Lymphozyten werden mit menschlichen Antiseren, die gegen bestimmte HL-Antigene gerichtet sind, und Komplement inkubiert. Komplement ist eine Serumkomponente und besteht aus einer Reihe von Proteinfaktoren, die sich gegenseitig in Form einer Enzymkaskade (vergleichbar dem Blutgerinnungssystem) aktivieren können. Der erste Komplementfaktor wird durch Bindung an einen Antigen-Antikörper-Komplex aktiviert. Ist das Antigen eine Zelle, in diesem Fall ein Lymphozyt, so führt der letzte Schritt der Komplementreaktion zur Lyse der Zelle.

Die für bestimmte HL-Antigene spezifischen menschlichen Antiseren werden von schwangeren Frauen gewonnen, die gewöhnlich Antikörper gegen die vom Vater vererbten HLA-Merkmale ihrer Kinder bilden.

Die bei erfolgter Immunreaktion durch Komplement lysierten Lymphozyten werden von den nichtlysierten Lymphozyten durch Anfärben mit einem Indikatorfarbstoff, wie z.B. Eosin, unterschieden. Das Ergebnis wird durch Betrachtung der Zellen im Phasenkontrastmikroskop ausgewertet.

Diese Methode hat wesentliche Nachteile: Die mikroskopische Bewertung des Ergebnisses ist weitgehend subjektiv und stellt eine Belastung für den Experimentator dar. Hierin liegt eine grosse Fehlermöglichkeit. Ein weiterer Nachteil ist, dass das Ergebnis in der Regel erst am nächsten Tag ausgewertet werden kann. Ausserdem ist der Test in dieser Form für eine Automatisierung nicht geeignet.

Aufgabe der Erfindung ist es nun, ein in der Handhabung einfaches, automatisierbares Verfahren zur HLA-Bestimmung zu schaffen, das eine objektivere und sicherere Aussage erlaubt

als die mikroskopische Bestimmung.

Gelöst wird diese Aufgabe erfindungsgemäss durch ein Verfahren zur Bestimmung von HL-Antigenen auf Lymphozyten durch Inkubation der Lymphozyten mit HLA-Antiserum und Komplement unter Freisetzung von ATP aus den durch Komplement lysierten Zellen, welches dadurch gekennzeichnet ist, dass man das freigesetzte ATP mit einer ATPase umsetzt, anschliessend die durch Komplement nicht lysierten Zellen rasch lysiert und das daraus freigesetzte ATP misst.

Es ist bekannt, dass bei der Zytolyse durch Komplement die Zellmembran für Metabolite durchlässig wird, so dass unter anderem auch ATP in das umgebende Medium austritt. Jedoch wird das ausgetretene ATP durch lymphozyteneigene ATPasen weitgehend abgebaut. Es war daher nicht zu erwarten, dass sich ein Test auf Basis der Messung von ATP für eine HL-Antigenbestimmung eignen würde.

Überraschenderweise wurde jedoch gefunden, dass in dem erfindungsgemässen Testsystem trotz Anwesenheit von ATPasen eine ATP-Bestimmung möglich ist.

Die Erfindung hat gegenüber der herkömmlichen mikroskopischen Methode folgende Vorteile:

1) Das Resultat ist objektivierbar, da es auf der Bestimmung eines messbaren Parameters (ATP) beruht und nicht der subjektiven Beurteilung des Experimentators unterliegt wie beim mikroskopischen Test.

2) Da alle Reaktionsschritte nur das Dosieren von Flüssigkeiten, den Transport von Lösungen und Inkubationen enthalten, ist das erfindungsgemässe Verfahren gut für eine Automatisierung geeignet.

3) Die Qualität der verwendeten Lymphozyten ist unkritisch. Auch ein Anteil toter Zellen, z.B. bei Leukämie, der bei der mikroskopischen Bestimmung zu erheblichen Fehlern führen würde, stört hier nicht.

Die Herstellung einer für den Test geeigneten Lymphozytensuspension kann nach den üblichen Methoden, wie z.B. durch Ficoll-Isopaque-Gradientenzentrifugation, erfolgen.

Die Inkubation der Lymphozyten mit Antiserum und Komplement wird in bekannter Weise nach der Methode des Zytotoxizitätstests (Terasaki, Kissmeyer-Nielsen) in einer geeigneten Pufferlösung durchgeführt. Geeignete Puffer sind Hanks' gepufferte Salzlösung (Proc. Exp. Biol. Med. 71, 196–200) oder Dulbecomedium (J. Expt. Med. 99, 167–182), pH 6,8 bis 7,4. Als Komplement wird bevorzugt Kaninchenkomplement verwendet.

Zum Abbau des aus den durch Komplement lysierten Zellen freigesezten ATP können alle bekannten ATP abbauenden Kinasen in Gegenwart eines Phosphatakzeptors verwendet werden, z.B. Adenosintriphosphatase EC 3.6.1.3 oder Apyrase EC 3.6.1.5 oder ATP-Pyrophosphatase EC 3.6.1.8. Bevorzugt wird aufgrund seiner grossen Stabilität das Enzym Apyrase EC 3.6.1.5, das z.B. aus Kartoffeln gewonnen werden kann, und die Reaktion $ATP + H_2O \rightarrow ADP + P_i$ katalysiert. Apyrase wird in einer Konzentration von 5 bis 2000 U/ml der Lmyphozytensuspension zugesetzt.

Zum Aufschluss der durch Komplement nicht lysierten Zellen eignen sich alle Methoden, die die Zellmembran rasch zerstören oder für Metabolite durchlässig machen. In einer bevorzugten Ausführungsform werden die Zellen mit Hilfe von Detergentien lysiert. Besonders bevorzugt werden nichtionische Detergentien, wie z.B. Polyäthylenoxidester von Alkyl- und Aralkylcarbonsäuren bzw. entsprechende Äther eingesetzt. Geeignete Konzentrationen an Detergens sind 0,05 bis 2%.

Die ATP-Bestimmung kann nach einem der bekannten Verfahren erfolgen (Bergmeyer «Methoden der enzymatischen Analyse», Bd. 2, S. 2147). Ein Beispiel dafür ist die Bestimmung mit 3-Phosphoglyceratkinase, der die folgenden Reaktionsgleichungen zugrunde liegen:

$$(1)\ \text{Glycerat-3-P} + \text{ATP} \xrightleftharpoons{\text{PGK}} \text{Glycerat-1.3-P}_2 + \text{ADP}$$

$$(2)\ \text{Glycerat-1.3-P}_2 + \text{NADH} + \text{H}^+ \xrightleftharpoons{\text{GAPDH}} \text{Glycerinaldehyd-3-P} + \text{NAD}^+ + \text{P}_i$$

PGK = 3-Phosphoglycerat-kinase EC. 2.7.2.3
GAPDH = Glycerinaldehyd-3-phosphat-dehydrogenase EC. 1.2.1.12

Eine weitere Möglichkeit ist die Bestimmung von ATP mit Hexokinase und Glucose-6-phosphat-dehydrogenase. Der Reaktionsablauf ist in den folgenden Gleichungen dargestellt:

$$(1)\ \text{ATP} + \text{Glucose} \xrightarrow{\text{HK}} \text{Glucose-6-phosphat} + \text{ADP}$$

$$(2)\ \text{Glucose-6-phosphat} + \text{NADP}^+ \xrightarrow{\text{G6P-DH}} \text{6-Phosphogluconsäure-}\delta\text{-lacton} + \text{NADPH} + \text{H}^+$$

HK = Hexokinase EC. 2.7.1.1
G6P-DH = Glucose-6-phosphat-dehydrogenase EC. 1.1.1.49

Eine weitere Möglichkeit ist die Bestimmung von ATP mit Formyltetrahydrofolat-Synthetase EC 6.3.4.3 nach folgenden Reaktionsgleichungen:

$$(1)\ \text{Formiat} + \text{ATP} + \text{Tetrahydrofolsäure} \rightleftharpoons \text{N}^{10}\text{-Formyltetrahydrofolsäure} + \text{ADP} + \text{Phosphat}$$

$$(2)\ \text{N}^{10}\text{-Formyltetrahydrofolsäure} \xrightleftharpoons{\text{H}^+} \text{5.10-Methenyltetrahydrofolsäure}$$

5,10-Methenyltetrahydrofolsäure besitzt ein Adsorptionsmaximum bei 350 nm und kann photometrisch durch Ermittlung der Extinktionszunahme bei dieser Wellenlänge bestimmt werden.

In einer bevorzugten Ausführungsform der Erfindung wird das freigesetzte ATP durch ein Biolumineszenzsystem, bestehend aus Luciferin und Luciferase bestimmt. Die Bestimmung beruht auf folgendem Prinzip:

$$(1)\ ATP + Luciferin \underset{Mg^{2+}}{\overset{Luciferase}{\rightleftharpoons}} Adenylyl\text{-}luciferin + Pyrophosphat$$

$$(2)\ Adenylyl\text{-}Luciferin \xrightarrow{O_2} Oxyluciferin + H_2O + Licht + CO_2 + AMP$$

Die freigesetzte Lichtmenge ist der umgesetzten ATP-Menge proportional und kann in einem vorbestimmten Zeitintervall mit einem Photometer gemessen werden. Der Lumineszenztest ist hochempfindlich und erfasst weniger als 10–13 mol ATP/l Versuchslösung.

Die ATP-Bestimmung wird in gepufferter Lösung durchgeführt. Geeignet sind alle Puffer, die in einem pH-Bereich zwischen 5 und 9, bevorzugt zwischen 6 und 8, puffern. Besonders bevorzugt werden Puffer, die die Konstanz des pH-Werts bei möglichst niedriger Ionenstärke aufrechtzuerhalten vermögen, wie z.B. Tris-Puffer, Hepes-Puffer und andere Goodpuffer.

Damit das freigesetzte ATP quantitativ erfasst werden kann, bevor ein nennenswerter Abbau durch ATPasen erfolgt ist, wird das ATP-Bestimmungssystem gleichzeitig mit dem Detergens, das die rasche Freisetzung des ATP bewirkt, der Lymphozytensuspension zugesetzt.

Ein weiterer Gegenstand der Erfindung ist ein Reagens zur Bestimmung des ATP der durch Komplement nicht lysierten Zellen, welches dadurch gekennzeichnet ist, dass es ein nichtionisches Detergens, Luciferin, Luciferase, Magnesium, Komplexierungsmittel, Apyrase, Serumalbumin und Puffer enthält. Die Reagenzkombination kann ausser den aufgeführten obligaten Bestandteilen zusätzlich übliche Lösungsmittel und Hilfsmittel, wie z.B. Stabilisatoren, enthalten.

Gemäss einer bevorzugten Ausführungsform enthält dieses Reagens

0,05 bis 2% nichtionisches Detergens,
0,1 bis 10 mg/l Luciferase,
15 bis 1000 µmol/l D-Luciferin,
2,5 bis 25 mmol/l Magnesiumchlorid,
0,05 bis 12,5 mmol/l EDTA
0,05 bis 12,5 g/l Rinderserumalbumin,
5 bis 2000 U/l Apyrase,
5 bis 100 mmol/l Puffer, pH 6 bis 8.

Besonders bevorzugt wird eine Ausführungsform, die 0,1 bis 1% Detergens, 2 bis 4 mg/l Luciferase, 40 bis 500 µmol/l D-Luciferin, 4 bis 12,5 mmol/l Magnesiumchlorid, 0,1 bis 1,3 mmol/l EDTA, 1 bis 6 g/l Rinderserumalbumin, 10 bis 100 U/l Apyrase, 10 bis 50 mmol/l Puffer enthält.

Die Anwesenheit weiterer Apyrase im erfindungsgemässen Reagens ist erforderlich, um das Untergrundsignal, das durch das Reagens allein verursacht wird, zu unterdrücken. Ohne die Anwesenheit von Apyrase ist eine ATP-Bestimmung bis zu einer Endkonzentration von etwa $10^{-11}$ mol/l ATP möglich. Die Anwesenheit von Apyrase im Reagens erhöht die Empfindlichkeit der Bestimmung auf $10^{-13}$ mol/l ATP.

Da die Apyrase im Reagens auch während der Biolumineszenzmessung das zu bestimmende ATP abbaut, muss die Messung in exakten Zeitintervallen erfolgen, um die Reproduzierbarkeit der Messung zu gewährleisten. In einer bevorzugten Ausführungsform der Erfindung wird mit der Lichtmessung spätestens 10 Sekunden nach Zusatz des erfindungsgemässen Reagens begonnen.

Ein hohes Messsignal zeigt an, dass unter den Reaktionsbedingungen keine komplementabhängige Zellyse auftrat und damit kein dem verwendeten Antiserum entsprechendes Antigen auf den Zellen vorhanden war. Ein niedriges Messsignal zeigt, dass eine Antigen-Antikörper-induzierte Komplement-bedingte Lyse auftrat, dass also in dem verwendeten System eine positive Reaktion stattfand.

Um das Messergebnis zu differenzieren, wird je eine Positiv- und eine Negativkontrolle mitbestimmt. Die Positivkontrolle entspricht 100%, die Negativkontrole 0% Lyse. Die Bewertung der Probe erfolgt nach dem Ausmass der Lyse: Eine Lyse $\geq$ 50% entspricht einer positiven Reaktion, eine Lyse $\leq$ 50% entspricht einer Kreuzreaktion und eine Lyse $<$ 20 bis 30% wird negativ bewertet. Eine Kreuzreaktion erhält man, wenn ein Antiserum mit mehr als einem bestimmten HL-Antigen reagiert. Z.B. findet man häufig Kreuzreaktionen zwischen HLA-A2 und HLA-A28 oder zwischen HLA-B7, HLA-B27 und HLA-BW22.

Die Tabelle zeigt bei fünf Probanden einen Vergleich zwischen der mikroskopischen Bestimmung und der ATP-Bestimmung im Biolumineszenztest. Die Übereinstimmung ist bis auf Bw6 bei Proband 4 vollständig. Hier könnte ein Fehler bei der mikroskopischen Ablesung vorliegen.

Tabelle
HLA-Bestimmung bei fünf Probanden; Vergleich der A) mikroskopischen Bestimmung mit B) der Biolumineszenzbestimmung

| | Proband 1 | | Proband 2 | | Proband 3 | | Proband 4 | | Proband 5 | |
|---|---|---|---|---|---|---|---|---|---|---|
| | A | B | A | B | | | | | | |
| | Eosin-färbung | Biolumi-neszenz % | Eosin-färbung | Biolumi-neszenz % | Eosin-färbung | Biolumi-neszenz % | Eosin-färbung | Biolumi-neszenz % | Eosin-färbung | Biolumi-neszenz % |
| A2 | – | – | – | – | – | – | + | 54 | + | 79 |
| A2+A28 | – | 32 | + | 72 | + | 100 | + | 100 | + | 100 |
| A9 | – | – | – | – | – | – | – | – | + | 42 |
| A25 | – | – | – | – | – | – | + | 100 | – | – |
| A25+A26 | – | – | – | – | – | – | + | 47 | – | – |
| B5 | – | 53 | – | – | – | – | – | – | – | – |
| B5+Bw35 | + | 100 | + | 89 | – | – | – | – | – | – |
| B7 | – | – | – | – | + | 100 | – | 56 | – | – |
| B8 | + | 92 | + | 47 | – | – | – | – | – | – |
| B12 | – | – | – | – | + | 60 | – | – | + | 60 |
| B13 | – | – | – | – | – | 33 | – | – | – | – |
| B14 | – | 27 | – | – | – | – | – | – | – | – |
| B15 | – | – | – | 35 | – | 38 | – | – | – | – |
| B17 | – | – | – | – | – | – | – | – | – | – |
| Bw49 | – | – | – | – | – | – | – | – | – | – |
| B27 | – | 29 | – | – | – | – | + | 93 | – | – |
| B37 | – | – | – | – | – | – | – | – | – | – |
| B40+B13 | – | – | – | – | – | – | – | – | – | – |
| Bw6 | + | 100 | + | 100 | + | 100 | – | 100 | + | 100 |
| Cw2 | – | – | – | – | – | – | + | 57 | – | – |

Die folgenden Beispiele erläutern die Erfindung weiter. Alle Biolumineszenzmessungen erfolgten mit dem Biolumat LB 9500 der Firma Laboratorium Prof. Berthold, Wildbad.

Beispiel 1

Herstellung eines Reagens zur Bestimmung von aus Lymphozyten freigesetztem ATP

1000 ml Reagens enthalten:
Hochgereinigte Luciferase aus Photinus pyralis 2,5 mg
D-Luciferin 450 µmol
Magnesiumchlorid 6,250 mmol
EDTA 0,625 mmol
Rinderserumalbumin 4 g
HEPES-Puffer 20 mmol, pH 7,75
Apyrase aus Kartoffeln 50 Einheiten.

Das Regens wird über Nacht im Dunkeln bei Raumtemperatur stehengelassen, damit das in den Reagenzien enthaltene ATP durch die Apyrase abgebaut werden kann. Da die Apyrase im Reagens während der Biolumineszenzmessung das zu bestimmende ATP sehr rasch abbaut, muss bei der Messung im exakten Zeittakt gearbeitet werden. Es wird z.B. zur Zeit 0 zu 400 µl Reagens 100 µl ATP enthaltender Probelösung gegeben und nach exakt 4,5 Sekunden für 10 Sekunden über die Lichtemission integriert. Auf diese Weise ist es möglich, verlässlich und reproduzierbar ATP-Konzentrationen bis $10^{-13}$ mol/l Endkonzentration zu bestimmen.

Dem Reagens wird kurz vor der im Beispiel 2 beschriebenen Messung Triton N-101 oder Triton X-100 in einer Endkonzentration von 0,1% zugegeben.

Beispiel 2

Test auf HL-Antigene

Es werden für die Messung die für den Biolumat passenden Kunststoffröhrchen verwendet. In diese Kunststoffröhrchen wird eine kleine Vertiefung von ∅ etwa 4 mm und einer Tiefe von 0,3 mm hineingebohrt. Die Röhrchen werden vor der Verwendung durch Vorbehandlung mit verdünnter Salzsäure von etwa vorhandenen ATP-Spuren befreit.

In die gebohrte Vertiefung werden zusammen pipettiert: 5 µl der zu testenden Lymphozytensuspension (500 Zellen/µl), 5 µl HLA-Antiserum.

Das Röhrchen wird mit einem Stopfen verschlossen und 30 Minuten bei Raumtemperatur inkubieren gelassen.

Dann werden zugegeben: 25 µl Kaninchenkomplement und 65 µl einer Apyraselösung (0,15 mU/l in 20 mmol/l Tris-Acetatpuffer, pH 7,75)

Die Röhrchen werden wieder verschlossen und mit dem Whirlmix wird die Lösung gut vermischt. Man lässt 60 Minuten bei Raumtemperatur stehen. Dann werden 400 µl detergenshaltiges ATP-Reagens zugegeben und die Messung unmittelbar darauf durchgeführt.

Bei einer positiven Reaktion des Antiserums werden die Lymphozyten in einem Ausmass von 50 bis 100% lysiert, und das aus den Zellen austretende ATP wird durch die Apyrase vollständig abgebaut. Mit dem ATP-Reagens wird hier im Idealfall nur ein Untergrundsignal erhalten.

Liegt eine negative Reaktion des Antiserums vor, bleiben die Lymphozyten im Laufe der Immunreaktion intakt. Sie werden dann durch das im Lumineszenzreagens enthaltene Detergens spontan quantitativ lysiert, das austretende ATP verursacht ein seiner Konzentration entsprechendes Lichtsignal.

Mit polyvalentem HLA-Antiserum wurde im Fall einer positiven Reaktion unter den hier beschriebenen Bedingungen beispielsweise ein Untergrundsignal von 15 000 Impulsen pro 10 Sekunden erhalten. Bei negativer Reaktion (negatives Vergleichsserum) wird hier ein Signal der Grösse 150 000 Impulse pro 10 Sekunden erhalten. Die Präzision dieser Signale ist mit einem VK von weniger als 10% so gut, dass sie eine sichere Unterscheidung zwischen positiver und negativer Reaktion erlaubt.

Beispiel 3

Herstellung von antiserumhaltigen Teströhrchen

Die Teströhrchen werden wie in Beispiel 2 beschrieben vorbereitet. Dann werden in die Vertiefung jeweils 5 µl Antiserum gegeben und lyophilisiert. Durch Zugabe von 5 bis 10 µl einer Lmyphozytensuspension entsprechenden Titers wird die Reaktion in Gang gesetzt und wie in Beispiel 2 beschrieben weiter durchgeführt.

Diese Variante bietet den Vorteil, dass eine grössere Anzahl von mit einem bestimmten Antiserum beschickten Röhrchen vorher bereitgestellt und gelagert werden können.

## Patentansprüche

1. Verfahren zur Bestimmung von HL-Antigenen auf Lymphozyten durch Inkubation der Lymphozyten mit HLA-Antiserum und Komplement unter Freisetzung von ATP aus den durch Komplement lysierten Zellen, dadurch gekennzeichnet, dass man das freigesetzte ATP mit einer ATP-ase umsetzt, anschliessend die durch Komplement nicht lysierten Zellen rasch lysiert, und das daraus freigesetzte ATP bestimmt.

2. Verfahren nach Anspruch 1, dadurch gekennzeichnet, dass man als ATPase Apyrase verwendet.

3. Verfahren nach Anspruch 1 oder 2, dadurch gekennzeichnet, dass man die durch Komplement nicht lysierten Zellen mit einem Detergens lysiert.

4. Verfahren nach Anspruch 1 bis 3, dadurch gekennzeichnet, dass man zur ATP-Bestimmung ein Luciferin/Luciferase-System zusetzt und die in einem vorbestimmten Zeitintervall emittierte Lichtmenge misst.

5. Verfahren nach Anspruch 1 bis 4, dadurch gekennzeichnet, dass die Lichtmessung spätestens 10 Sekunden nach Zusatz des Luciferin/Luciferase-Systems beginnt,.

6. Reagens zur Bestimmung des ATP der durch Komplement nicht lysierten Zellen, dadurch gekennzeichnet, dass es ein nichtionisches Detergens, Luciferin, Luciferase, Magnesium, Komplexierungsmittel, Apyrase, Serumalbumin und Puffer enthält.

7. Reagens nach Anspruch 6, dadurch gekennzeichnet, dass es
0,05 bis 2% nichtionisches Detergens,
0,1 bis 10 mg/l Luciferase,
15 bis 1000 µmol/l D-Luciferin,
2,5 bis 25 mmol/l Magnesiumchlorid,
0,05 bis 12,5 mmol/l EDTA,
0,05 bis 12,5 g/l Rinderserumalbumin,
5 bis 2000 U/l Apyrase und
5 bis 100 mmol/l Puffer, pH 6 bis 8,
enthält.

## Claims

1. Process for the determination of HL antigens on lymphocytes by incubation of the lymphocytes with HLA antiserum and complement, with the liberation of ATP from the cells lysed by complement, characterised in that one reacts the liberated ATP with an ATPase, subsequently rapidly lyses the cells not lysed by complement and determines the ATP liberated therefrom.

2. Process according to claim 1, characterised in that one uses apyrase as ATPase.

3. Process according to claim 1 or 2, characterised in that one lyses with a detergent the cells not lysed by complement.

4. Process according to claims 1 to 3, characterised in that, for the ATP determination, one adds a luciferin/luciferase system and measures the amount of light emitted in a predetermined time interval.

5. Process according to claims 1 to 4, characterised in that the light measurement commences at most 10 seconds after addition of the luciferin/luciferase system.

6. Reagent for the determination of the ATP of the cells not lysed by complement, characterised in that it contains a non-ionic detergent, luciferin, luciferase, magnesium, complexing agent, apyrase, serum albumin and buffer.

7. Reagent according to claim 6, characterised in that it contains
0.05 to 2% non-ionic detergent,
0.1 to 10 mg/l luciferase,
15 to 1000 µmole/l D-luciferin,
2.5 to 25 mmole/l magnesium chloride,
0.05 to 12.5 mmol/l EDTA,
0.05 to 12.5 g/l bovine serum albumin,
5 to 2000 U/l apyrase and
5 to 100 mmol/l buffer, pH 6 to 8.

## Revendications

1. Procédé de détermination des HL-antigènes sur des lymphocytes par incubation des lymphocytes avec du HLA-antisérum et du complément avec libération d'ATP à partir des cellules lysées par le complément, caractérisé en ce qu'on fait réagir l'ATP libéré avec une ATPase, puis on lyse rapidement les cellules non lysées par le complément, et on détermine l'ATP libéré de celles-ci.

2. Procédé suivant la revendication 1, caractérisé en ce qu'on utilise comme ATPase de l'apyrase.

3. Procédé suivant la revendication 1 ou 2, caractérisé en ce qu'on lyse avec un détergent les cellules non lysées par le complément.

4. Procédé suivant la revendication 1 à 3, caractérisé en ce que pour la détermination de l'ATP, on ajoute un système luciférine/luciférase et on mesure la quantité de lumière émise dans un intervalle de temps prédéterminé.

5. Procédé suivant la revendication 1 à 4, caractérisé en ce que la mesure de la lumière commence au plus tard 10 secondes après addition du système luciférine/luciférase.

6. Réactif pour la détermination de l'ATP des cellules non lysées par le complément, caractérisé en ce qu'il contient un détergent non ionique, de la luciférine, de la luciférase, du magnésium, de l'agent complexant, de l'apyrase, de la sérumalbumine et du tampon.

7. Réactif suivant la revendication 6, caractérisé en ce qu'il contient:

0,05 à 2% de détergent non ionique,
0,1 à 10 mg/l de luciférase,
15 à 1000 µmoles/l de D-luciférine,
2,5 à 25 mmoles/l de chlorure de magnésium,
0,05 à 12,5 mmoles/l d'EDTA,
0,05 à 12,5 g/l de sérumalbumine de bœuf,
5 à 2000 U/l d'apyrase et
5 à 100 mmoles/l de tampon, pH 6 à 8.